# EUROPEAN PATENT SPECIFICATION

(11) **EP 3 424 541 B1**
(45) Date of publication and mention of the grant of the patent: **24.12.2025**
(21) Application number: 17759106.2
(22) Date of filing: 13.02.2017
(51) Int. Cl.: A61L 27/36, A61L 27/16, A61L 27/18, A61L 27/22, A61L 27/58, A61L 27/50

(54) **COMPOSITE SOFT TISSUE REPAIRING MATERIAL FOR STABILIZING REPAIR REGION**
ZUSAMMENGESETZTES WEICHGEWEBEREPARATURMATERIAL ZUR STABILISIERUNG DER REPARATURREGION
MATÉRIAU COMPOSITE DE RÉPARATION DE TISSU MOU DESTINÉ À STABILISER UNE RÉGION DE RÉPARATION

(30) Priority: 01.03.2016 CN 201610115203
(43) Date of publication of application: 09.01.2019
(73) Proprietor: Shanghai Zhuoruan Medical Technologies Co., Ltd, Shanghai 200233 (CN); Zhuoruan Medical Technologies (Suzhou) Co., Ltd, Taicang, Jiangsu 215434 (CN)
(72) Inventor: ZHANG, Jian, Shanghai 200433 (CN)
(74) Representative: Epping - Hermann - Fischer
(86) International application number: PCT/CN2017/073395
(87) International publication number: WO 2017/148255

(56) References cited:
- CN-A- 104 822 342
- CN-A- 105 664 257
- US-A1- 2010 185 219
- US-A1- 2014 099 330
- US-A1- 2015 209 478
- US-A1- 2015 352 145
- B. MA ET AL: "Crosslinking strategies for preparation of extracellular matrix-derived cardiovascular scaffolds", REGENERATIVE BIOMATERIALS, vol. 1, no. 1, 1 November 2014 (2014-11-01), pages 81 - 89, XP055544473, ISSN: 2056-3418, DOI: 10.1093/rb/rbu009
- ZHANG: "Additional Experimental Data E1 - Shanghai Zhuiruan Medical Technologies Co., Ltd", 24 May 2022 (2022-05-24), pages 1 - 6, XP93062058, Retrieved from the Internet <URL:http://citenpl.internal.epo.org/wf/web/citenpl/citenpl.html> [retrieved on 20220524]

## Description

### TECHNICAL FIELD

The present invention is defined by the appended claims and relates to the field of soft tissue repair, and particularly to a composite soft tissue repair material stable in repair area.

### BACKGROUND OF THE INVENTION

Soft tissue repair and reconstruction for abdominal wall defects, chest wall defects, dura mater defects, and burn wounds are important topics in surgery and regenerative medicine, in which repair for large defects is a difficult problem in surgery.

In recent years, application of various synthetic materials including polypropylene patches, polyester patches, expanded polytetrafluoroethylene patches, and polyglycolic acid patches has realized tension-free repair, thereby reducing the recurrence rate. In the repair process of synthetic materials, under the chronic inflammation stimulation of synthetic fibers, inflammatory cells, fibroblasts, and collagen from the host proliferate into the mesh of non-degradable patches to form a scar tissue with "steel bar-concrete" structure, and the long-term mechanical strength of the repair area is maintained well. However, the non-degradable fiber is a long-term chronic inflammatory stimulus to the host, and complications such as patch discharge, chronic pain, intestinal adhesion, intestinal obstruction, and even intestinal fistula still occur sometimes. These complications often lead to more complicated surgical problems that require higher treatment costs and also hernia formation/recurrence. Susceptibility to bacteria implantation as well as the resulting chronic infection is another potential problem for the synthetic materials. The non-degradable synthetic materials are good carriers for the bacteria. Once the bacteria adhere to the synthetic materials, biofilms will form, which is difficult to be eliminated by the immune system of the body, even if the dose of antibiotics is increased by 1000 times. Further, some synthetic materials such as expanded polytetrafluoroethylene patches have defects such as incapability to fuse with host tissues and formation of fibers capsulations around the material after implantation, which are less resistance to infection, and must be removed in case of infection.

Acellular matrix biologic patches are composed of allogeneic or xenogeneic extracellular matrix (e.g., submucosa of hollow viscera) or inert tissue matrix (e.g., dermis, pericardium, and peritoneum). The matrix components are derived from tissues in which all cells, antigens, lipids, soluble proteins, etc. are removed using a chemical detergent along with other auxiliary reagents, having an intact appearance and histological and ultra-microstructure, mainly including collagen, elastin, proteoglycan, glycosaminoglycan, and non-collagen glycoprotein. Therefore, the acellular matrix biologic patches can maintain the same appearance of the tissue, provide a scaffold for cell regeneration, and also stimulate and induce the infiltration of cells or collagen of the host to repair tissue defects. Meanwhile, since the cell components causing immune responses are removed, the immune rejection can be avoided. The acellular matrix biologic patches can be completely absorbed and utilized by the body and exert functions through host tissue remodeling.

However, the long-term efficacy of the acellular matrix biologic patches is still controversial, mainly because the repair areas of some cases experience laxity, eventration (Bulge), or even hernia recurrence, especially when complicated and large abdominal wall defect is repaired using the biologic patches by bridging method. Some studies have found that more than 50% of the cases with abdominal wall bulging in repair area one year after surgery, and severe cases are therefore surgically implanted with synthetic patches to reinforce the repair area. As for the existing precautions, only some manufacturers suggest that in use of the biologic patches such as an acellular dermal patch, the patch should be stretched as much as 50% of the original area after the material is hydrated, and then used for defect repair, because the incidence of long-term abdominal wall bulging will increase without stretching. A long-term research has found that the long-term loss of laxity in repair area of the biologic patches is due to the simultaneous biodegradation accompanying with remodeling (endogenous biological healing). If degradation of the implant material does not balance with the new tissue remodeling, the tension will not be supported or transmitted entirely and timely, resulting in loss of laxity, bulging or hernia recurrence in the repair area of the biological patches.

In order to allow the implanted patches-new tissue undergo a smooth transfer of tissue tension, the researchers have considered introducing non-degradable components or slowly degradable components into biologic patches. CN201664349U discloses a novel composite hernia and body wall repair patch. The patch is composed of polymer mesh, bio-derived material, medical adhesive to adhere polymer mesh to bio-derived material. The composite hernia repair patch can effectively isolate the direct contact between the abdominal organs and the synthetic mesh. The lightweight mesh can play the role of "steel bar" in the repair area in the long term, optimize the overall tensile performance of the composite mesh, significantly reduce the recurrence of hernia, and improve the hand feeling.
(1) The polymer mesh covers the surface of the acellular matrix and directly contacts with host tissues after implantation, changing the immune response type in the host-material margin of the acellular matrix (M2-type macrophage infiltration in dominance → M1-type macrophage infiltration in dominance).
(2) Most biological materials are applied to the contaminated wounds, and the bacteria are easy to adhere to polymer material. Therefore, after the bacterial adhesion, biofilms can be formed that protect the bacteria from host immune defense and antibiotics, so that the bacteria can grow and proliferate locally for a long time and cause chronic infection of wounds.

CN102698318A discloses a biomaterial composite patch. The patch is composed of synthetic material as base layer, and extracellular matrix/decellularized tissue matrix material biological patch, or bio-synthetic material patch or other synthetic degradable material as a supplemental layer. The base layer and the supplemental layer are assembled by medical suture or medical glue or other methods. The basic mechanism and structure of the patch are the same as that of CN201664349U. In addition to the above-mentioned problems such as the tissue exposure to the polymer material, severe inflammatory reaction and no infection resistance, the patch has the following problems.
(1) The description about anti-adhesion and resistance to infection mentioned in the SUMMARY OF THE INVENTION is inaccurate. Biological materials have a structure similar to that of natural tissues, and the biological materials used alone are resistant to infection to some extent, while combined with a synthetic patch, infection will be inevitably causes since the synthetic patch is exposed to the tissue of the body, so that the composite material is not resistant to infection as a whole material.
(2) The polymer material as a base layer will experience the same adhesion as a synthetic material.
(3) Standard-weight, non-absorbable mesh may be used when a polymer material is used as base layer. Then there may be problems such as poor compliance to the abdominal wall, and strong foreign body sensation in the repair area during application.
(4) The patch is assembled by a base layer with specific size and a supplemental layer under sterile conditions during surgery. The preparation is time consuming and labor intensive, the patch is inconvenient to use, and the stability of the structure cannot be guaranteed.

Further attention is drawn to US2014099330, disclosing a tissue prosthesis comprising a support structure having at least one surface, the support structure comprising a base material, the support structure further including an extracellular matrix (ECM) composition having at least one ECM material from a mammalian tissue source.

In addition, in US2010185219 there is presented a composite material for use in a medical application, comprising a biological material and a reinforcement material.

### SUMMARY OF THE INVENTION

The technical issue the present invention aims to solve is to provide a composite soft tissue repair material stable in repair area, which combines the advantages of crosslinked materials and non-crosslinked materials, including high histocompatibility, no viscera erosion, resistance to infection, mechanical stability of the repair area or the like, and thus has good application prospects.

The present invention is set forth in the appended claims.

A composite soft tissue repair material stable in repair area of the present invention includes a middle mechanical reinforcement layer containing a crosslinked material; and the upper layer and the lower layer containing a non-crosslinked membrane-like acellular matrix, wherein the upper layer and the lower layer completely encapsulate the middle mechanical reinforcement layer to form a sandwich structure, and the tensile strength of the middle mechanical reinforcement layer is > 16 N/cm, and wherein the crosslinked material contains certain amounts of a non-absorbable component, and the crosslinked material is derived from acellular matrix.

The crosslinked material is encapsulated by the non-crosslinked membrane-like acellular matrix, rather than apply to the surface of the non-crosslinked membrane-like acellular matrix. The non-crosslinked membrane-like acellular matrix isolates the direct contact with the synthetic material or crosslinked material with the host tissue after implantation, does not change the immune response type in host-material margin of the composite material as a whole, and avoids colonization of the bacteria to non-absorbable components.

An edge of the middle mechanical reinforcement layer is 2 mm to 15 mm away from an edge of the upper layer and the lower layer. This ensures that no mechanical reinforcement layer is exposed to the surface of the composite material. The non-crosslinked membrane-like acellular matrix acts as surface layers to endow the composite material with high histocompatibility and capability to direct contact with the organs. A tensile strength for the middle mechanical reinforcement layer is > 16 N/cm (the natural human abdominal fascia strength), and an elasticity thereof is slightly lower than the natural human abdominal fascia strength, and the middle mechanical reinforcement layer is mechanically stable in a long term.

A mass ratio of synthetic material or crosslinked material in the middle mechanical reinforcement layer to the non-crosslinked membrane-like acellular matrix in the upper and lower layers is 1:20 to 1:1, so as to ensure that non-degradable components of relatively low contents can compensate for the instability of the repair area caused by poor tissue regeneration induced by the non-crosslinked membrane-like acellular matrix in special cases.

The crosslinked material contains certain amounts of a non-absorbable component, such as a mesh or a membrane made of a polymer material or a metal material; or a crosslinked membrane-like acellular matrix. The middle mechanical reinforcement layer can improve the bending resistance and hand feeling as a whole, and the crosslinked membrane-like acellular matrix can be used as a mechanical reinforcement layer to achieve significant mechanical improvement while reducing stiffness as a whole.

The non-absorbable polymer material comprises one or more medical polymers such as polypropylene, polyester, polyvinylidene fluoride, and silicone; the non-absorbable metal material comprises one or two of titanium alloy and nickel titanium alloy; also disclosed is that a slowly absorbable polymer material comprises one or more of poly-4-hydroxybutyric acid, polylactic acid, polyglycolic acid, polytrimethylene carbonate, glycolide/lactide/polytrimethylene carbonate, and fibroin; and the slowly absorbable metal material comprises degradable magnesium alloy.

In the mesh made of the non-absorbable polymer material, a mass of the polymer material is less than 60 g/m², and preferably less than 40 g/m², and a voids diameter is more than 3 mm.

The crosslinked membrane-like acellular matrix is derived from submucosa of hollow organs, dermis, pericardium, peritoneum, pleura, basement membrane, or amniotic membrane of human or mammalian; with a crosslinking degree from 1% to 100%. The crosslinking degree is variable, but the long-term stable mechanical properties thereof should meet the requirements of the middle mechanical reinforcement layer.

The non-crosslinked membrane-like acellular matrix is derived from submucosa of hollow organs, dermis, pericardium, peritoneum, pleura, or amniotic membrane of human or mammalian.

The crosslinked membrane-like acellular matrix or the non-crosslinked membrane-like acellular matrix may be single-layered or multi-layered structures, and the numbers of layers should be changed depending on the position of use.

The middle mechanical reinforcement layer and the upper and lower layers are laminated by one or more of medical adhesive, tying with suture, and vacuum pressing.

Preferably, the medical adhesive and the suture are composed of absorbable components. The vacuum pressure of the vacuum pressing is -13332.236535 Pa (-100 mmHg) to - 101324.997664 Pa (-760 mmHg), and the acting duration is 0.5 h to 72 h.

The composite soft tissue repair material is further perforated penetrating the material, wherein the perforations have a diameter of 1 mm to 5 mm, and the spacing between perforations is 1 mm to 5 mm, and no synthetic material or crosslinked material exposed in the perforations. In the case of the mechanical reinforcement layer comprises microporous mesh, the composite soft tissue repair material does not perforate through the mesh fibers, and the perforations locate in the voids of the mechanical reinforcement layer.

The crosslinking in the present invention refers to intramolecular or intermolecular covalent bonding between collagen molecules or between the collagen molecules and other components in the non-crosslinked membrane-like acellular matrix by physical or chemical means, so as to block the targets of collagenases. Therefore, the resulted matrix will not be degraded or only partially degraded after implantation, and has similar or even better biomechanical properties than the synthetic mesh, thereby achieving the purpose of permanent reserve of part of the implanted undegradable fiber and stability of the repair area.

### EFFECTS OF THE INVENTION

(1) The repair area of the present invention is stable in the long-term, without laxity, bulging or shrinking. In the early stage of repair, the three-dimensional collagen network of the acellular matrix of the upper and lower layers of the patch can induce rapid ingrowth of host cells and blood vessels. The non-degradable components ensure that the lowest point of the mechanical properties of remodeled acellular matrix meets the needs of tissue repair and smoothly completes the transmission of tension. In the middle and late stage of repair, the acellular matrix is remolded and combined with the non-absorbable/permanent components implanted to maintain the tension-free repair of tissue defects for a long time, and finally the repair area achieves a long-term stable structure similar to "steel bar-concrete". Even under special circumstance, such as unsatisfied induced regeneration of central area in large-area defect repair, and the non-degradable crosslinked material can continue providing a certain mechanical tension to avoid the loss of elasticity and tissue bulging in the repair area. Further, the non-absorbable mesh in the repair area has an ultra-lightweight and macroporous structure, and the repair area will not shrink in the long term.
(2) Good histocompatibility. The upper and lower layers of the repair material of the present invention contains non-crosslinked acellular matrix components. The repair material has good histocompatibility, can directly contact organs such as the intestine, with minimal organ adhesion and without severe host reaction. Further, non-absorbable polymer fibers are completely encapsulated in the non-crosslinked acellular matrix layers, and the edge of which is 2 mm to 15 mm away from the edge of upper and lower layers (the suture margin of surgery is generally 5 mm, and the distance of the present invention can ensure suturing to the mechanical reinforcement layer), which can ensure that crosslinked collagen fibers cannot directly contact the surrounding tissues in the early stage of implantation. The degradation of xenogeneic acellular matrix is basically synchronous with the ingrowth of host tissue, thus effectively keeps the organs isolate from the non-absorbable fibers, preventing the fibers from eroding tissues. The perforations penetrating the whole "sandwich" structure accelerate the ingrowth of surrounding fibrous tissues, which facilitate the flow of tissue fluid, so that the incidence of local complications such as seroma is lowered. Even if the upper and lower layer is completely degraded to expose the crosslinked material, viscera erosion and shrinkage will not occur because the crosslinked material is derived from the acellular matrix, which is mainly composed of collagen and is totally different from the synthetic material.
(3) Resistance to infection. In view that the biologic patch currently is mainly applied in contaminated or infected wound repair, the mechanical reinforcement layer introduced should meet the following two requirements: avoid bacterial colonization at the early stage after implantation. Non-absorbable fibers are completely encapsulated in the acellular matrix layers. Whether at the edge of the material or in the perforations, the fibers are difficult to directly contact the bacteria. The synthetic mesh of single-strand, ultra-macroporous and ultra-lightweight is difficult to hide bacteria. Therefore, as a whole, resistance to infection of the "sandwich" structure should be similar to acellular matrix biologic patch. Further, the acellular matrix is mainly composed of type I and type III collagen fibers, with components such as laminin and glycosaminoglycan. The matrix is of high porosity and negatively charged, so that the positively charged anti-infective particles such as nano-silver particles, can be easily adhere to the matrix. Thus, anti-infective components in the adhered matrix can be continuously released to the surrounding extracellular matrix after implantation. Therefore, the repair material of "sandwich" structure can be easily developed into a material equipped with the ability of infective resistance with broad antibacterial spectrum, effectiveness and long-lasting time.
(4) High strength. Two layers of acellular matrix can provide at least 2 times higher mechanical strength than the human abdominal fascia, which can meet the load requirements during and after wound healing, and the middle mechanical reinforcement layer can also provide a certain degree of tension. The tensile strength of the composite material is expected to be more than 3 times than that of natural abdominal fascia.
(5) Low amounts of permanent implants and no influence on abdominal wall compliance. In the "sandwich" material, the mass ratio of the non-absorbable components in the middle mechanical reinforcement layer to the non-crosslinked membrane-like acellular matrix in the upper and lower layers is less than or equal to 1:1. Since the non-absorbable fibers is not the major force-supporting components in the early stage, but mainly function as a role of "steel bar" in the repair area in the long-term, no large scars are formed and patients' comfort and flexibility will be improved after surgery.
(6) Good memory and handling feel. The vacuum pressing and tying with suture avoid delamination of the material, making it easy for the surgeon to cut the material into suitable shapes according to the wounds during the operation. Further, the middle mechanical reinforcement layer imparts modified elasticity and flexibility to the material for the surgeon to use under laparoscopy.

### BRIEF DESCRIPTION OF THE DRAWINGS

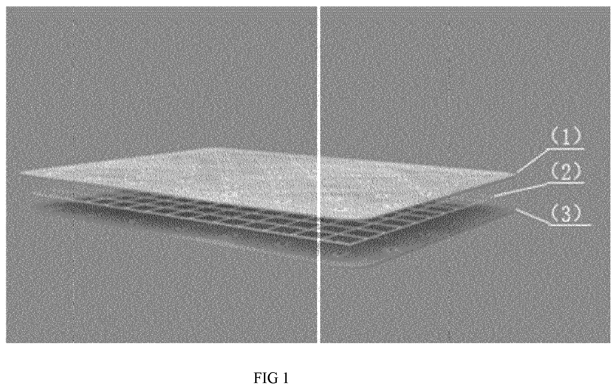

FIG. 1 is a schematic structural view of the present invention, wherein (2) is the middle mechanical reinforcement layer, and (1) and (3) are upper and lower layers.

### DETAILED DESCRIPTION OF THE PREFERED EMBODIMENT

The present invention will be further illustrated below in conjunction with specific embodiments. It should be understood that these embodiments are only used to illustrate the present invention and are not intended to limit the scope of the present invention.

### Example 1 (not part of the invention)

As shown in FIG. 1, a non-crosslinked porcine small intestinal submucosa (SIS) was prepared by Abraham method. Membrane-like SIS was spliced into larger-area layer by an interlacing method, and three layers were laid and bonded as a whole by a medical chitosan adhesive, namely non-crosslinked membrane-like acellular matrix layer. The non-crosslinked membrane-like acellular matrix layer was perforated with voids spacing as 5mm, diameter as 1mm. A macroporous polypropylene mesh had voids spacing of 5×5 mm and a mass of 40 g/m². The non-crosslinked membrane-like acellular matrix was placed as an upper layer (1) and a lower layer (3), and the microporous polypropylene mesh was placed as a middle mechanical reinforcement layer (2) to form a "sandwich" structure, in which the mass ratio of the non-crosslinked membrane-like acellular matrix to the macroporous polypropylene mesh was 10:1. The edges of the polypropylene mesh sheet was 5 mm away from the edges of the non-crosslinked membrane-like acellular matrix, respectively. Make sure that no polypropylene fiber was exposed to the perforations of the non-crosslinked membrane-like acellular matrix. The three layers were bonded by a medical chitosan adhesive, pressed into a whole by a pressure of -200 mmHg for 18 h, and then perforated with spacing of 5mm and diameter of 1mm. The material was perforated with voids spacing as 5mm, diameter as 1mm.

According to the standard GB/T528-2009 of China, three samples were taken and cut into a dumbbell shape of 4 cm × 1 cm. Two ends of the patch were fixed by a material mechanical tester and stretched at a speed of 10 mm/min to measure the tensile strength of the material to be 52±8 N/cm.

An animal model of canine rectus abdominis anterior sheath and rectus abdominis muscle defect with local hypertension (a water bladder was added between the rectus abdominis posterior sheath and the repair material) was constructed, in which the defect area was 10 × 5 cm². The SIS-PP composite patch was cut into a certain size for repairing the animal model. After surgery, the tension of the water bladder was increased to 40 mmHg by water injection month by month. For 24 months of observation, there was no abdominal wall bulging in the repair area. The tensile strength of the implanted patch was measured to be 32 ± 8 N/cm.

### Example 2

As shown in FIG. 1, membrane-like small intestinal submucosa (SIS) was prepared by Abraham method. Membrane-like SIS sheets were spliced into a larger-area layer in an interlacing manner, and three layers were laid and bonded as a whole by a medical chitosan adhesive. The material was perforated with voids spacing as 5mm, diameter as 1mm, namely non-crosslinked membrane-like acellular matrix. High-strength crosslinked membrane-like SIS of was prepared by treating non-crosslinked SIS with 0.5% (w/v) genipin solution for 72 hours, crosslinking degree of which was 50% by ninhydrin method and weight was 38g/m². Non-crosslinked membrane-like acellular matrix layers as an upper layer (1) and a lower layer (3) and high-strength crosslinked membrane-like acellular matrix as the middle mechanical reinforcement layer (2) were placed to form a "sandwich" structure, in which the mass ratio of the crosslinked membrane-like acellular matrix in the middle mechanical reinforcement layer to the non-crosslinked membrane-like acellular matrix in the layers was 2:1. The edge of the middle mechanical reinforcement layer was 5 mm away from the edge of the non-crosslinked acellular, which ensured that no crosslinked material was exposed in the perforations of the 10 acellular matrix. The three layers were bonded by a medical chitosan adhesive.

According to the standard GB/T528-2009 of China, Tensile strength of the material was measured to be 50 ± 5 N/cm.

An animal model of canine rectus abdominis anterior sheath and rectus abdominis muscle defect with local hypertension (a water bladder was added between the rectus abdominis posterior sheath and the repair material) was constructed, in which the defect area was 10 × 5 cm². The material was cut into a certain size for repairing the animal model. After surgery, the tension of the water bladder was increased to 40 mmHg by water injection month by month. For 24 months of observation, there was no abdominal wall bulging in the repair area. The tensile strength of the implanted patch was measured to be 30 ± 5 N/cm.

## Claims

1. A composite soft tissue repair material stable in repair area, comprising:
a middle mechanical reinforcement layer containing a crosslinked material;
an upper layer containing a non-crosslinked membrane-like acellular matrix; and
a lower layer containing a non-crosslinked membrane-like acellular matrix,
wherein the upper layer and the lower layer completely encapsulate the middle mechanical reinforcement layer to form a sandwich structure, and
the tensile strength of the middle mechanical reinforcement layer is > 16N/cm, and
wherein the crosslinked material contains certain amounts of a non-absorbable component, and the crosslinked material is derived from acellular matrix.

2. The composite soft tissue repair material stable in repair area according to claim 1, wherein an edge of the middle mechanical reinforcement layer is 2 mm to 15 mm away from an edge of the upper layer and an edge of the lower layer.

3. The composite soft tissue repair material stable in repair area according to claim 1, wherein a mass ratio of the crosslinked membrane-like acellular matrix in the middle mechanical reinforcement layer to the non-crosslinked membrane-like acellular matrix in the upper layer and the lower layer is 1:20 to 1:1.

4. The composite soft tissue repair material stable in repair area according to claim 3, wherein the crosslinked membrane-like acellular matrix is derived from submucosa of hollow organs, dermis, pericardium, peritoneum, pleura, or amniotic membrane of human or mammalian;
wherein a number of layers of the crosslinked membrane-like acellular matrix is 1 to 10, and
wherein a crosslinking degree is 1% to 100%.

5. The composite soft tissue repair material stable in repair area according to claim 1, wherein the non-crosslinked membrane-like acellular matrix is derived from submucosa of hollow organs, dermis, pericardium, peritoneum, pleura, or amniotic membrane of human or mammalian.

6. The composite soft tissue repair material stable in repair area according to claim 1, wherein the middle mechanical reinforcement layer, the upper layer and the lower layer are laminated by one or more of medical adhesive, tying with suture, and vacuum pressing.

7. The composite soft tissue repair material stable in repair area according to claim 1, wherein the material is further perforated penetrating the material, wherein the perforations have a diameter of 1 mm to 5 mm, wherein the spacing between perforations is 1 mm to 5 mm, wherein no crosslinked material exposed in the perforations.

## Patentansprüche

1. Kompositweichgewebe-Reparaturmaterial, das im Reparaturbereich stabil ist, umfassend:
eine mittlere mechanische Verstärkungsschicht, die ein vernetztes Material enthält;
eine obere Schicht, die eine nicht vernetzte membranartige azelluläre Matrix enthält; und
eine untere Schicht, die eine nicht vernetzte membranartige azelluläre Matrix enthält,
wobei die obere Schicht und die untere Schicht die mittlere mechanische Verstärkungsschicht vollständig einkapseln, um eine Sandwichstruktur zu bilden, und
die Zugfestigkeit der mittleren mechanischen Verstärkungsschicht > 16N/cm ist, und
wobei das vernetzte Material bestimmte Mengen einer nicht absorbierbaren Komponente enthält und das vernetzte Material von einer azellulären Matrix abgeleitet ist.

2. Kompositweichgewebe-Reparaturmaterial, das im Reparaturbereich stabil ist, gemäß Anspruch 1, wobei eine Kante der mittleren mechanischen Verstärkungsschicht 2 mm bis 15 mm von einer Kante der oberen Schicht und einer Kante der unteren Schicht entfernt ist.

3. Kompositweichgewebe-Reparaturmaterial, das im Reparaturbereich stabil ist, gemäß Anspruch 1, wobei das Massenverhältnis der vernetzten membranartigen azellulären Matrix in der mittleren mechanischen Verstärkungsschicht zu der nicht vernetzten membranartigen azellulären Matrix in der oberen Schicht und der unteren Schicht 1:20 bis 1:1 beträgt.

4. Kompositweichgewebe-Reparaturmaterial, das im Reparaturbereich stabil ist, nach Anspruch 3, wobei die vernetzte membranartige azelluläre Matrix aus Submukosa von Hohlorganen, Dermis, Perikard, Peritoneum, Pleura oder Amnionmembran von Menschen oder Säugetieren gewonnen wird;
wobei die Anzahl der Schichten der vernetzten membranartigen azellulären Matrix 1 bis 10 beträgt, und
wobei ein Vernetzungsgrad von 1 % bis 100 % vorliegt.

5. Kompositweichgewebe-Reparaturmaterial, das im Reparaturbereich stabil ist, gemäß Anspruch 1, wobei die nicht vernetzte membranartige azelluläre Matrix aus Submukosa von Hohlorganen, Dermis, Perikard, Peritoneum, Pleura oder Amnionmembran von Mensch oder Säugetier stammt.

6. Kompositweichgewebe-Reparaturmaterial, das im Reparaturbereich stabil ist, gemäß Anspruch 1, wobei die mittlere mechanische Verstärkungsschicht, die obere Schicht und die untere Schicht durch einen oder mehrere der folgenden Verfahren laminiert sind: medizinischer Klebstoff, Binden mit Naht und Vakuumpressen.

7. Kompositweichgewebe-Reparaturmaterial, das im Reparaturbereich stabil ist, gemäß Anspruch 1, wobei das Material weiterhin das Material durchdringend perforiert ist, wobei die Perforationen einen Durchmesser von 1 mm bis 5 mm haben, wobei der Abstand zwischen den Perforationen 1 mm bis 5 mm beträgt, wobei kein vernetztes Material in den Perforationen freiliegt.

## Revendications

1. Matériau composite de réparation de tissus mous, qui est stable dans une zone de réparation, contenant :
une couche de renfort mécanique centrale contenant un matériau réticulé ;
une couche supérieure contenant une matrice acellulaire non-réticulée de type membrane ; et
une couche inférieure contenant une matrice acellulaire non-réticulée de type membrane,
la couche supérieure et la couche inférieure entourant complètement la couche de renfort mécanique centrale afin de former une structure en sandwich, et
la résistance à la traction de la couche de renfort mécanique centrale étant supérieure à 16 N/cm, et
le matériau réticulé contenant des quantités déterminées d'un composant non résorbable et le matériau réticulé étant obtenu à partir d'une matrice acellulaire.

2. Le matériau composite de réparation de tissus mous, qui est stable dans une zone de réparation, selon la revendication 1, sachant qu'un bord de la couche de renfort mécanique centrale est distant de 2 mm à 15 mm d'un bord de la couche supérieure et d'un bord de la couche inférieure.

3. Le matériau composite de réparation de tissus mous, qui est stable dans une zone de réparation, selon la revendication 1, sachant que le rapport massique entre la matrice acellulaire réticulée de type membrane dans la couche de renfort mécanique centrale et la matrice acellulaire non réticulée de type membrane dans la couche supérieure et la couche inférieure est compris entre 1:20 et 1:1.

4. Le matériau composite de réparation de tissus mous, qui est stable dans une zone de réparation, selon la revendication 3, sachant que la matrice acellulaire réticulée de type membrane est obtenue à partir de la sous-muqueuse d'organes creux, du tissu sous-cutané, du péricarde, du péritoine, de la plèvre ou de la membrane amniotique d'êtres humains ou de mammifères ;
le nombre de couches de la matrice acellulaire réticulée de type membrane étant compris entre 1 et 10, et
le degré de réticulation étant compris entre 1 % et 100 %.

5. Le matériau composite de réparation de tissus mous, qui est stable dans une zone de réparation, selon la revendication 1, sachant que la matrice acellulaire non réticulée de type membrane est obtenue à partir de la sous-muqueuse d'organes creux, du tissu sous-cutané, du péricarde, du péritoine, de la plèvre ou de la membrane amniotique d'êtres humains ou de mammifères.

6. Le matériau composite de réparation de tissus mous, qui est stable dans une zone de réparation, selon la revendication 1, sachant que la couche de renfort mécanique centrale, la couche supérieure et la couche inférieure sont laminées à l'aide d'un adhésif médical et/ou par liaison à l'aide d'un matériel de suture et/ou par pressage sous vide.

7. Le matériau composite de réparation de tissus mous, qui est stable dans une zone de réparation, selon la revendication 1, sachant que le matériau est en outre perforé et ainsi pénétré, les perforations ayant un diamètre de 1 mm à 5 mm et la distance entre les perforations étant de 1 mm à 5 mm, aucun matériau réticulé n'étant exposé dans les perforations.
